# EUROPEAN PATENT APPLICATION

(11) **EP 4 625 392 A1**
(43) Date of publication of application: **01.10.2025**
(21) Application number: 25164727.7
(22) Date of filing: 19.03.2025
(51) Int. Cl.: G09B 5/12, G09B 7/04, G09B 19/00

(54) **INFORMATION PROCESSING METHOD, PROGRAM, INFORMATION PROCESSING DEVICE, AND INFORMATION OUTPUT SYSTEM**

(30) Priority: 25.03.2024 JP 2024047454; 23.12.2024 JP 2024226000
(71) Applicant: CASIO COMPUTER CO., LTD., Tokyo 151-8543 (JP)
(72) Inventor: Ueda, Masashi, Hamura-shi, Tokyo 205-8555 (JP); Yoshida, Nobuto, Hamura-shi, Tokyo 205-8555 (JP); Ono, Ryunosuke, Hamura-shi, Tokyo 205-8555 (JP); Shimizu, Hiroshi, Hamura-shi, Tokyo 205-8555 (JP); Ishii, Katsunori, Hamura-shi, Tokyo 205-8555 (JP); Azuma, Yunosuke, Hamura-shi, Tokyo 205-8555 (JP); Tomita, Hiroki, Hamura-shi, Tokyo 205-8555 (JP)
(74) Representative: Grünecker Patent- und Rechtsanwälte PartG mbB

(57) **Abstract**

An information processing method to be performed by a computer (4, 7, 4a, 7a) includes: obtaining a target period for goal achievement of a user; setting a goal, based on the target period and current ability of the user; and determining a practice method, based on the goal and the target period.

## Description

### TECHNICAL FIELD

The present disclosure relates to an information processing method, a program, an information processing device, and an information output system.

### DESCRIPTION OF RELATED ART

The training of athletes is usually managed by trainers and adjusted to effectively enhance their ability. For ordinary people, a known technology links daily exercise to training management of a fitness club (Japanese Patent Application No. 2018-45393).

### SUMMARY OF THE INVENTION

However, people mostly perform practice (learning) on their own without receiving external advice, based on magazines, information on the Internet, and word-to-mouth information to improve their ability, such as physical fitness (e.g., running and jogging), skills, and academic ability. There are also cases where people simply read texts or do workbooks given by educational institutions. People who practice on their own often struggle to gauge their level of improvement in relation to their practice. Accordingly, these people may not improve their ability as they wish or may not reach their goals.

An object of the present disclosure is to provide an information processing method, a program, an information processing device, and an information output system that can provide effective practice methods to users.

According to the present disclosure, there is provided an information processing method to be performed by a computer, including: obtaining a target period for goal achievement of a user; setting a goal, based on the target period and current ability of the user; and determining a practice method, based on the goal and the target period.

### Advantageous effect of the invention

According to the present disclosure, an effective practice method can be provided to a user.

### BRIEF DESCRIPTION OF DRAWINGS

FIG. 1 illustrates a configuration of an information output system according to an embodiment.
FIG. 2A is a block diagram illustrating a functional configuration of an electronic device.
FIG. 2B is a block diagram illustrating a functional configuration of a server device.
FIG. 3 illustrates the flow of determining a strategy for creating a practice plan.
FIG. 4 is a flowchart of the control procedure of a practice plan creating process.
FIG. 5A is a flowchart of the control procedure of a candidate goal extracting process to be executed in the practice plan creating process.
FIG. 5B is a flowchart of the control procedure of a practice method extracting process.
FIG. 6 illustrates a configuration of an information output system according to a second embodiment.
FIG. 7A is a block diagram illustrating a functional configuration of an electronic device in the second embodiment.
FIG. 7B is a block diagram illustrating a functional configuration of a server device in the second embodiment.
FIG. 8A is a chart showing contents of data regarding determination and execution of a learning method.
FIG. 8B is a chart showing contents of data regarding determination and execution of the learning method.
FIG. 8C is a chart showing contents of data regarding determination and execution of the learning method.
FIG. 8D is a chart showing contents of data regarding determination and execution of the learning method.
FIG. 9A illustrates an example of the display screen when a learning plan is created.
FIG. 9B illustrates an example of the display screen when the learning plan is created.
FIG. 9C illustrates an example of the display screen when the learning plan is created.
FIG. 9D illustrates an example of the display screen when the learning plan is created.

### DETAILED DESCRIPTION

Hereinafter, embodiments of the present disclosure are described with reference to the figures.

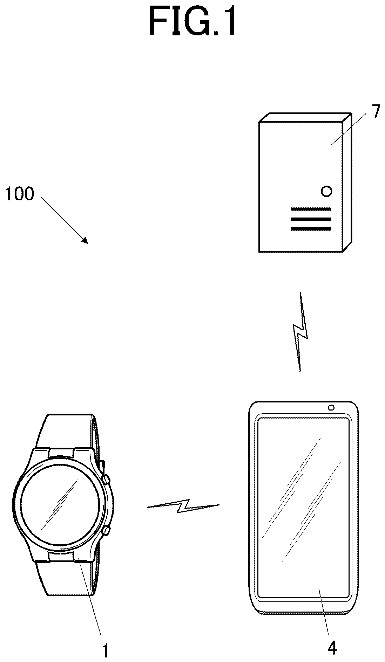

An information output system 100 of a first embodiment shown in FIG. 1 makes a plan for a user to achieve a goal in an activity, such as running. The information output system 100 includes a measurement device 1, an electronic device 4, and a server device 7.

The measurement device 1 is a body-worn terminal device that is worn on the user's body to perform measurement on the activity. Herein, the measurement device 1 is a wristwatch-type device, such as a smart watch or an activity meter. There may be two or more measurement devices 1. For example, there may be a waist-worn measurement device 1 in addition to the wristwatch-type device. The measurement device 1 receives manipulations related to selection, start, and stop of the activity and performs measurement. The measurement device 1 can display simple measurement information and so forth in real time during measurement and after measurement.

The electronic device 4 obtains measurement results by the measurement device 1 and analyzes the measurement results. The electronic device 4 can display advice, comments, and so forth based on the analysis results, together with the analysis results. The electronic device 4 is, for example, a smartphone. The measurement device 1 and the electronic device 4 may be constantly connected for a long time for communications. For example, the measurement device 1 and the electronic device 4 may be communicatively connected by the low energy standard of Bluetooth (registered trademark).

The server device 7 obtains analysis results from the electronic devices 4 of users and stores the analysis results in a database 732. The server device 7 performs statistical processing of the analysis results stored in the database 732 and obtains information on a large number of users, such as the distribution of ability levels and the tendency of ability improvements by training. The electronic device 4 of each user can refer to these pieces of information, based on the level of the user, for example. The electronic device 4 is communicatively connected to the server device 7 via the Internet. For the communication connection, a wireless LAN and/or a mobile phone line may be used, for example.

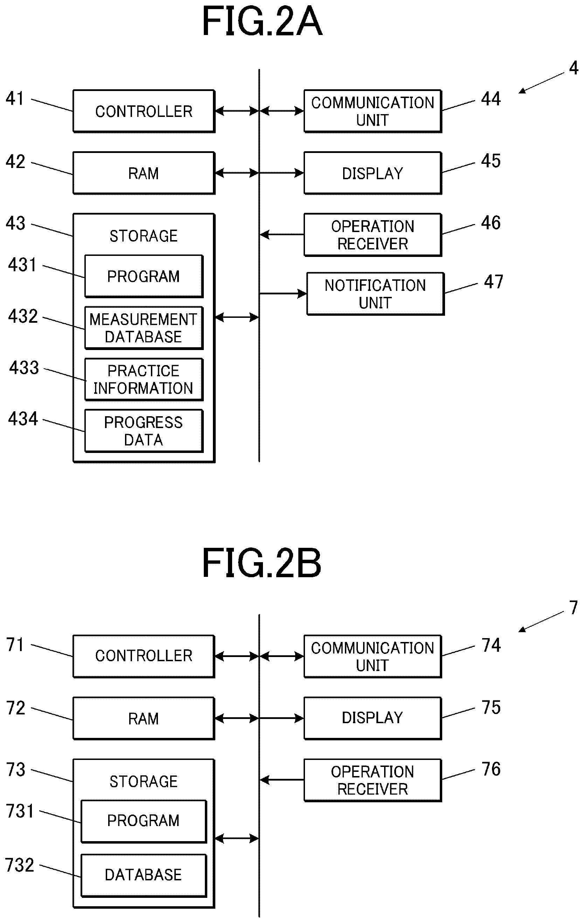

As illustrated in the block diagram of FIG. 2A, the electronic device 4 includes a controller 41, a random access memory (RAM) 42, a storage 43, a communication unit 44, a display 45, an operation receiver 46, and a notification unit 47.

The controller 41 includes a processor that controls overall operations of the electronic device 4. The processor may be a general-purpose central processing unit (CPU) or a dedicated microcomputer. A single processor may centrally perform entire processing, or multiple processors may perform processing in parallel or independently for their respective purposes.

The RAM 42 provides a working memory space for the processor of the controller 41 and stores temporary data. The RAM 42 may be, for example, a DRAM. The temporary data includes measurement results during the activity obtained from the measurement device 1. The measurement results may be deleted after the analysis process ends.

The storage 43 is a nonvolatile memory. The storage 43 stores a program 431, setting data, measurement database 432, and so forth. The measurement database 432 includes analysis results of measurement results in the past activity. The nonvolatile memory may be a flash memory, for example. The setting data includes practice information 433 and plan progress data 434 regarding the activity. The program 431 may include individual programs of a practice plan creating process, a goal candidate extracting process, and a practice method extracting process, which are described later.

The communication unit 44 includes a module for controlling communication operations. As described above, the communication unit 44 can be communicatively connected to the measurement device 1 over Bluetooth and can communicate with the server device 7 over the Internet, for example. The communication unit 44 may obtain weather information around the current location from outside, especially information on rainfall, snowfall, wind direction, and wind strength, for example.

The display 45 includes a digital display. Under the control of the controller 41, the display 45 controls display contents on the digital display screen. The display 45 may also be configured to display information on functions other than information on the activity.

The operation receiver 46 includes, for example, a touchscreen and a push button switch. The touchscreen is overlaid on the digital display screen. The touchscreen detects a touch operation and outputs a signal indicating the touched position to the controller 41. The operation receiver 46 detects a press of the push button switch and outputs a detection signal to the controller 41.

The notification unit 47 performs notification to the outside. The notification may be generation of a beep, generation of vibration, or output of sounds, for example. The notification unit 47 has a configuration corresponding to the type of notification.

As illustrated in the block diagram of FIG. 2B, the server device 7 may be a normal personal computer (PC), for example. The server device 7 includes a controller 71, a RAM 72, a storage 73, a communication unit 74, a display 75, and an operation receiver 76.

The controller 71 includes a processor that controls overall operations of the server device 7. The processor may be a general-purpose CPU or a dedicated microcontroller. A single processor may centrally perform entire processing, or multiple processors may perform processing in parallel or independently for their respective purposes.

The RAM 72 provides a working memory space for the processor of the controller 71 and stores temporary data. The RAM 72 may be, for example, a DRAM.

The storage 73 is a nonvolatile memory and stores a program 731, a database 732, and so forth. The nonvolatile memory may be, for example, a hard disk drive (HDD) or a flash memory. The storage 73 may be an auxiliary storage device externally attached to the main body of the PC. For another example, the storage 73 may include a network drive or a cloud server provided on a network.

The communication unit 74 includes a module that controls communication operations. As described above, the communication unit 74 can communicate with the electronic device 4 over the Internet.

The display 75 includes a digital display. Under the control of the controller 71, the display 75 controls display contents on the digital display screen.

The operation receiver 76 may include a push button switch, a pointing device such as a mouse, and a keyboard, for example. The operation receiver 76 converts input operations on these components into electrical signals and outputs the signals to the controller 71.

The controller 71, the RAM 72, the storage 73, and the communication unit 74 constitute the PC (computer) main body. The display 75 and the operation receiver 76 may be peripheral devices attached to the PC main body. For another example, the server device 7 may be basically accessed via an external terminal device, and the display 75 and so forth except the power button, the reset switch, and so forth may not be normally connected to the server device 7.

The database 732 stores profiles of a large number of users, practice methods (strategies) used by the large number of users in the past, implementation periods of the methods, performance records corresponding to the implementation of the methods, changes in parameters, and so forth. Herein, a method refers to a determined way, method, or scheme for achieving a goal and/or object. The database 732 also stores basic information of methods, such as: goal levels, required practice frequencies, and required time and loads corresponding to the respective methods; and outlines of detailed plans corresponding to the methods. Examples of a practice method include Practice method A (technique, physical, mental), Practice method B (technique, physical, tactics), and Practice method C (technique, physical, mental, tactics). A practice method may include items other than the above items (technique, etc.) defined for each method. The database 732 may also store statistical information, such as improvement rates of ability by methods, achievement rates of goals, and rates of cases where the methods were stopped (given up) halfway. In some methods, the level of practice is increased step by step. Users who have reached a certain level of ability may start practice from the corresponding level. Therefore, the information on a method may include ability of the user at the time of adopting the method in association with the implementation time of the method.

Following is the description of making a plan for the user to achieve his/her goal in running.

The information processing method of this embodiment determines a range of realistic goals, based on the user's current ability and the target date (deadline) for achieving the goal. The information processing method also determines an effective practice method toward the determined goal. Based on the determined method, detailed practice plans are determined.

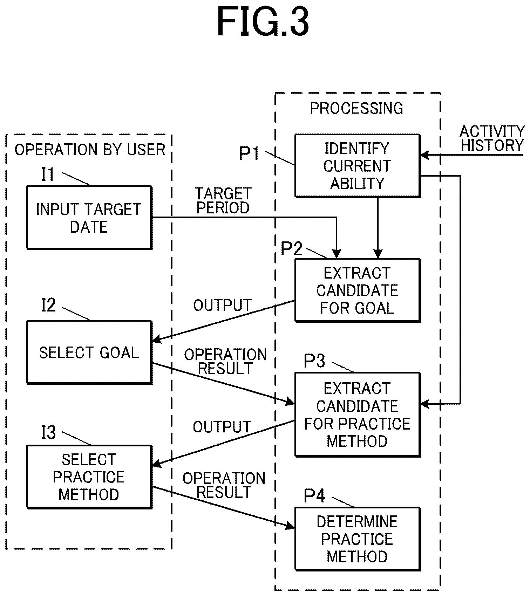

As shown in FIG. 3, the current ability of the user is identified from the activity history of the user so far (P1). The activity history includes information on the distance that the user runs and the running time (speed) in usual practice. Based on the information, the ability of the user can be identified. The activity history is the accumulation of data obtained by the measurement device 1, integrally processed by the electronic device 4, and analyzed by the server device 7.

Users often set his/her goal, based on competitions and tournaments. Therefore, in this embodiment, the user firstly inputs and sets the deadline for achieving the goal (I1). A goal that is achievable within days (target period) by the deadline is determined, based on the current ability. Then, a strategy of plans effective for achieving the goal is determined.

In this embodiment, candidates for the goal and candidates for the practice strategy corresponding to the above conditions are selected from the database 732 and presented to the user as recommendations (P2, P3). There may be one or more recommendations. The user approves or selects a recommendation, so that the goal and the practice strategy are determined (I2, I3). Based on the determined practice strategy, practice plans are drafted (P4).

The information processing apparatus of this embodiment that performs processing of extracting recommendations of the goal and the practice strategy may be the electronic device 4 or the server device 7. When the electronic device 4 performs the above processing, the server device 7 extracts necessary information registered in the database 732 and sends the information to the electronic device 4. When the server device 7 performs the above processing, the server device 7 sends information on recommendations for the user (display data) to the electronic device 4, and the electronic device 4 sends information regarding input operations by the user to the server device 7. In the following, the server device 7 performs the processing.

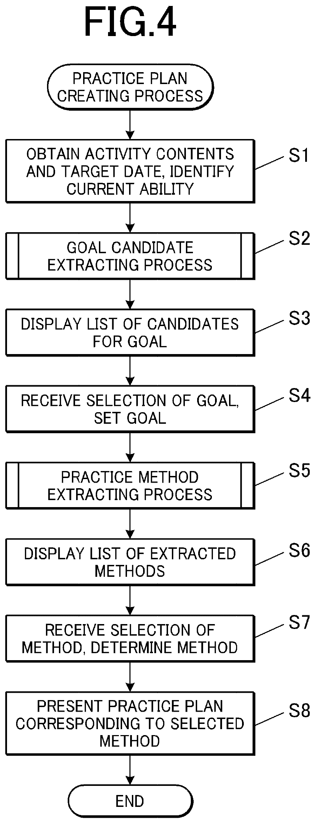

The practice plan creating process of this embodiment in FIG. 4 starts when the server device 7 receives a request for creating plans from the electronic device 4 and executes the program 731. When the operation receiver 46 of the electronic device 4 obtains the deadline, the electronic device 4 sends the request for creating plans along with the deadline and the user identification information to the server device 7.

The controller 71 obtains activity contents of the user and the deadline (S1: obtaining means). The controller 71 obtains the activity contents of the user by searching the database 732, based on the user identification information. The activity contents may include information on the running speed and the running distance in practice and performance (e.g., competitions) and information on the frequency of the activity, mainly the frequency of practice. Based on the deadline and the current date, a target period during which the user can perform practice for achieving the goal is obtained. The controller 71 identifies the current ability (actual ability) of the user, based on the practice contents (also referred to as the practice history) of the user so far, mainly based on the amount of practice. The practice contents may include analysis results obtained from the electronic device 4 (information on the movement of the user's body in running, such as part or all of indicator values of a stride, a pitch, a tilt or sway of the upper body, the magnitude of arm swing, the rotation of the hip position, and the magnitude of left/right sway and up/down movements, for example).

The controller 71 executes the candidate goal extracting process, which is described later (S2). The controller 71 obtains a candidate(s) for the goal (candidate goal) that exceeds the current ability within an achievable range, based on the identified current ability (actual ability). Multiple candidate goals may be obtained.

The controller 71 displays the obtained candidate goals as a list on the display 75 or sends, to the electronic device 4 via the communication unit 74, control data for displaying the list on the display 45 of the electronic device 4 (S3). The controller 71 waits for data that indicates contents of input operations on the operation receiver 76 or data that indicates contents of input operations on the operation receiver 46 via the communication unit 74. The controller 71 obtains the result of the input operations as the selection result. In accordance with the obtained operation result, the controller 71 determines the goal to be achieved (S4: determining means).

The controller 71 executes the practice method extracting process to obtain candidates for the practice method (practice method candidates) corresponding to the determined goal (S5). Multiple practice method candidates may be extracted.

The controller 71 displays the obtained practice method candidates as a list on the display 75 or outputs, to the electronic device 4 via the communication unit 74, control data for displaying the list on the display 45 of the electronic device 4 (S6). When there are multiple practice method candidates, the display or communication data is controlled such that the candidates are displayed as a list in the order described later. The candidate practice methods may be displayed along with their overviews, required practice time and load, the number of times the respective methods have been selected, the achievement rate in the selected times, and so forth.

The controller 71 waits for data that indicates contents of input operations on the operation receiver 76 or data that indicates contents of input operations on the operation receiver 46 via the communication unit 74. The controller 71 obtains the result of the input operations as the selection result. Based on the obtained operation result, the controller 71 determines the practice method (S7: determining means). Even when there is only one candidate for the practice method, the controller 71 obtains contents of input operations or the result of input operations by the user indicating whether the user approves the candidate.

The controller 71 creates and outputs a practice plan, based on the determined practice method (S8). The controller 71 may output the practice plan to the display 75 or the display 45 for display or may send the practice plan to the electronic device 4 as a document file. The controller 71 then ends the practice plan creating process.

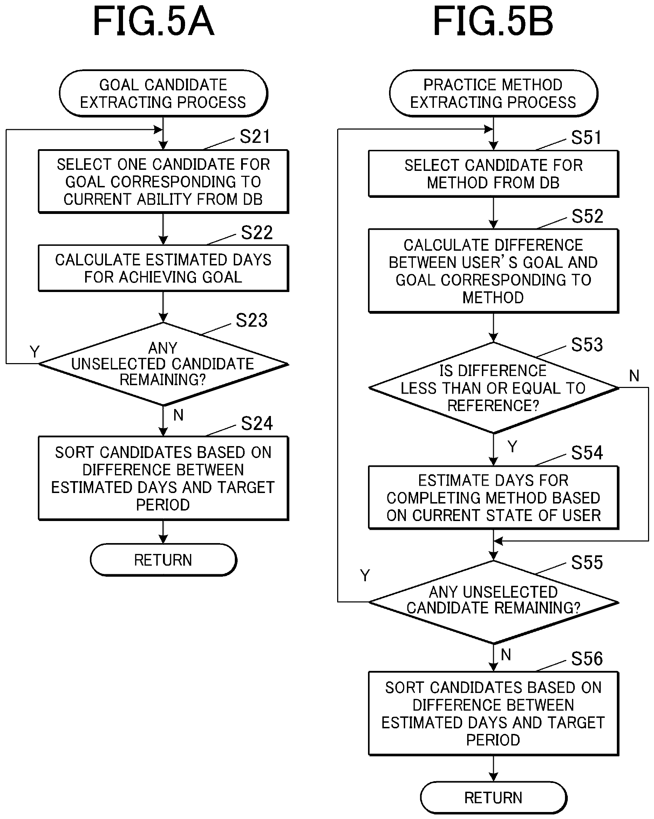

As shown in the candidate goal extracting process in FIG. 5A, the controller 71 selects one candidate goal based on the state of the user (e.g., current ability) from the database 732, which the controller 71 is referring to (S21). The controller 71 sequentially obtains one combination from combinations of a range of running distance and a range of running time stored in the database 732. The database 732 may simply store a range of running time that can be set for each running distance and steps that can be set in the range of running time. For example, in a case of a full marathon (42.195 km), the database 732 may store information that the goal can be set in 5-minute increments between 2 hours 30 minutes and 5 hours 30 minutes.

The controller 71 calculates the estimated number of days as the estimated period until the user achieves the goal, based on the user's ability and the difference between the user's ability and the selected goal (S22). Naturally, the estimated number of days depends on the practice method described later and the practice frequency. Therefore, the controller 71 may output an average value among past users at the same level, for example. The estimated number of days corresponding to a candidate goal that is below the user's current ability may be zero (0), a negative value, or a value indicating an error.

The controller 71 determines whether there is any unselected candidate goal (S23). When determining that there is an unselected candidate goal (S23: Y), the controller 71 returns to Step S21. When determining that there is no unselected candidate goal (S23: N), the controller 71 arranges (sorts) the candidate goals, based on the difference between the estimated number of days and the target period (S24).

As described above, candidate goals having the estimated number of days of "0", a negative value, or a value indicating an error may be excluded. The controller 71 may exclude candidate goals having the difference greater than a predetermined upper limit. Herein, the difference having a positive value may indicate that the user is estimated to achieve the goal in a period shorter than the target period. The remaining candidate goals may be arranged in ascending order of absolute values of the difference. For another example, the controller 71 may prioritize candidate goals having positive values or negative values as the difference in arranging them. The criteria for the arrangement may be determined beforehand by the user's operations, for example. For another example, the criteria for the arrangement may be adjusted according to the selection tendency of the user in the past. The values indicating the difference may be deleted after the arrangement or may be stored in association with the corresponding candidate goals. The controller 71 then ends the candidate goal extracting process and returns to the practice plan creating process.

As shown in FIG. 5B, in the practice method extracting process, the controller 71 selects one candidate for the practice method from the database 732 (S51). The controller 71 calculates the difference between the goal of the user and the range of goal levels corresponding to the candidate for the practice method (S52). Herein, the goal levels need to be expressed in scalar values for comparison. The weight per unit time of the difference between the running time as the user's goal and the running time corresponding to the goal level of the practice method depends on the running distance. Therefore, the magnitude of the difference can simply be the difference in time per unit distance (i.e., the difference in speed). Further, a weight corresponding to the running distance may be applied to the difference in time.

Further, the goal of the user and the goal of the practice method may be expressed in a two-dimensional coordinate system of running distance and running time normalized with their respective weights. The difference between the goal of the user and the goal of the practice method may be obtained as the Euclidean distance or the Mahalanobis' distance. In practice, the difference expressed by the Mahalanobis' distance is more likely to be accurate because the speed depends on the running distance. The difference may be between the user's level and the level closest to the user's level in the range of goal levels corresponding to the practice method. For another example, the difference may be between the user's level and a representative value of the range. Also, the difference may be a positive or negative real number. For example, the plus/minus sign may be attached, based on whether components corresponding to the running time among the above are large or small.

The controller 71 determines whether or not the calculated difference is less than or equal to a reference value (S53). When the difference is the shortest value between the user' s level and the range of goal levels as described above, the reference value may be "0" or a small positive value. When the difference is between the user's level and the representative value of the range, the reference value may be an appropriate positive value. For another example, the reference value may be determined beforehand, based on setting operations by the user, for example.

When determining that the difference is neither less than nor equal to the reference value (S53: N), the controller 71 proceeds to step S55. When determining that the difference is less than or equal to the reference value (S53: Y), the controller 71 calculates the estimated time as the estimated period for the user to complete practice of the selected method and achieve the goal, based on the current ability of the user (S54). Herein, the estimated time may be expressed in the number of days. The current practice frequency and practice time of the user may also be considered in calculating the estimated time. Specifically, in a case where the user currently performs practice relatively frequently or for a relatively long time, the calculated estimated time may be shorter than in a case where the user performs practice relatively less frequently or for a relatively short time. The estimated time may be calculated in consideration of the user's improvement rate of ability during a certain period. The controller 71 then proceeds to step S55.

In step S55, the controller 71 determines whether any unselected candidate practice method remains (S55). When determining that an unselected candidate practice method(s) remains (S55: Y), the controller 71 returns to step S51.

When determining that no unselected candidate practice method remains (S55: N), the controller 71 arranges the candidate practice methods according to the difference between the target period and the number of days (estimated period), which has been calculated for each of the candidate practice methods (S56). The values expressing the difference may be stored in association with the corresponding candidate methods or may be deleted after the candidate methods are arranged. The controller 71 may arrange the candidate methods in ascending order of absolute values of the difference. For another example, in sorting, the controller 71 may prioritize the candidate methods having positive values as the difference, namely the candidate methods with which the user is estimated to achieve the goal in a period shorter than the target period. Conversely, in sorting, the controller 71 may prioritize the candidate methods having negative values as the difference, namely the candidate methods with which the user is estimated to achieve the goal in a period longer than the target period. The criteria for the arrangement may be determined beforehand by the user's operations, for example. For another example, the criteria for the arrangement may be adjusted according to the selection tendency of the user in the past. The controller 71 then ends the practice method extracting process and returns to the practice plan creating process.

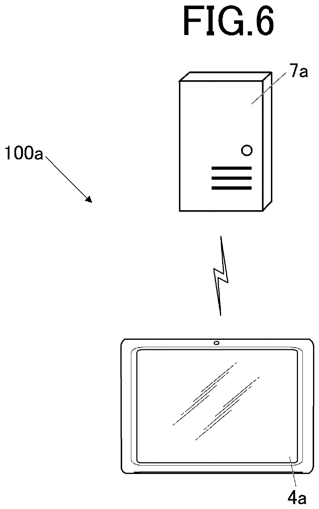

In a second embodiment, an information output system 100a illustrated in FIG. 6 makes a plan for the user to achieve his/her goal in learning and provides data for learning to the user. The information output system 100a includes an electronic device 4a and a server device 7a. The electronic device 4a may be a terminal device, such as a tablet or a notebook PC, or a desktop PC.

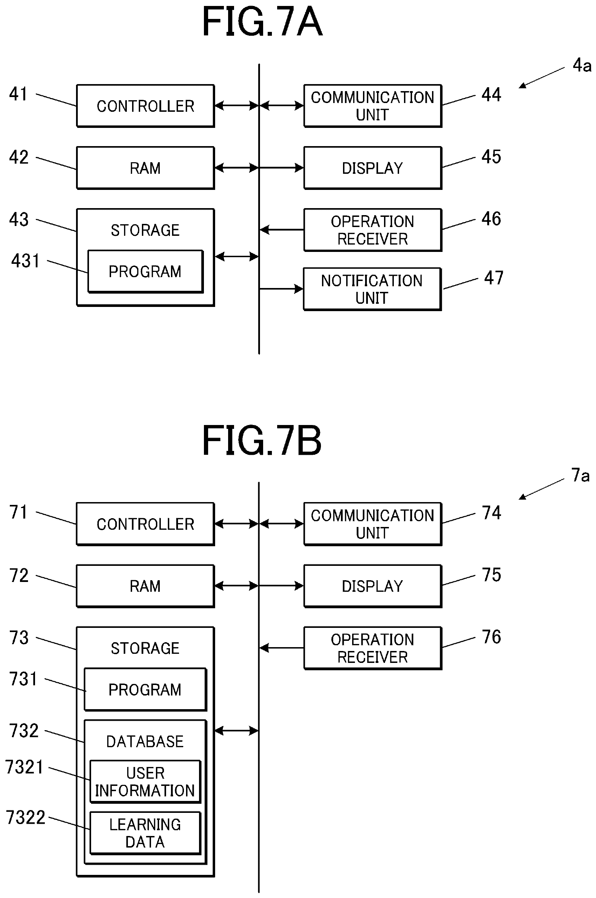

In the information output system 100a of the second embodiment, the server device 7a may store all the information for managing performance of the user and learning contents to be provided to the user. That is, the electronic device 4a may simply display contents and receive operations. As shown in FIG. 7A, the electronic device 4a has a program 431 in the storage 43. The program 431 outputs operation signals based on inputs by the user.

As shown in FIG. 7B, the database 732 of the server device 7a in this embodiment stores user information 7321 and learning data 7322. The user information 7321 stores learning settings and learning progress states of the respective users. The user information 7321 may include statistical data on the learning progress states and performance information of multiple users. The learning data 7322 stores learning contents to be provided to users, namely texts, questions, and answers, for example. The learning data 7322 may be provided by a third party. The texts and questions are each associated with various learning methods. That is, each text, question, and answer is read out and sent to the electronic device 4a when the user reaches a certain step in the order set by the learning method selected by the user. The data that has been provided to the user once may be re-provided at the user's request as needed. The learning data 7322 may store requirements necessary to complete each subject or unit and solve each question.

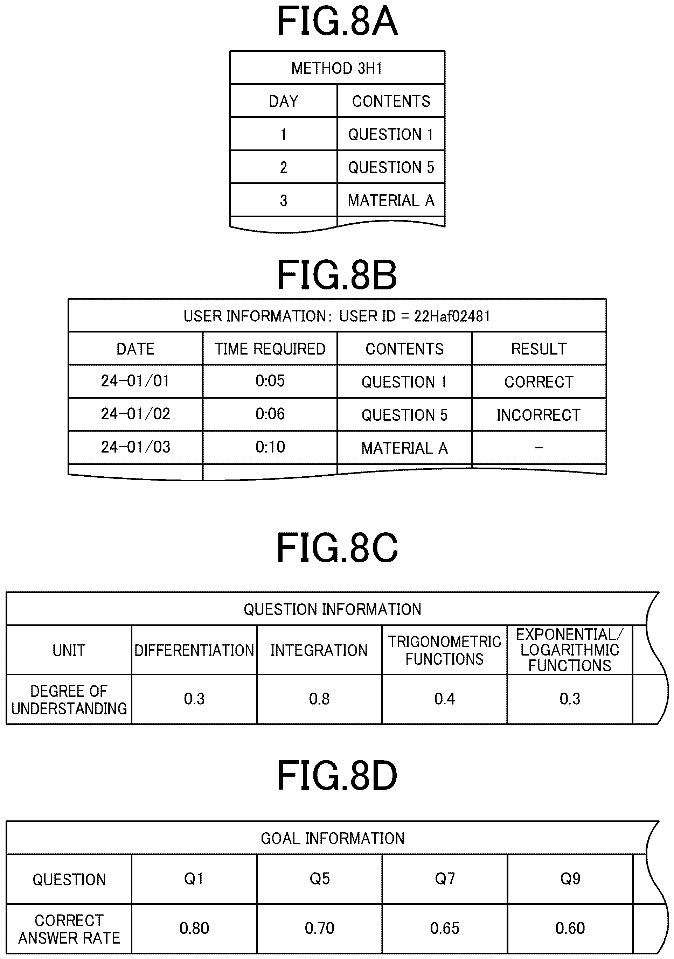

As shown in FIG. 8A, in the learning method, contents to be provided to the user are arranged by date. The contents include texts and questions as described above. The user may be able to obtain answers by sending responses to the provided questions. For questions with clear answers, the answer of the user may be determined to be correct or wrong. For questions that require essay-type answers, a simple determination may be made on whether the answer of the user includes necessary terms; or optionally, a marker who belongs to the operation side of the server devices 7a may grade the answer on a later date.

The procedure of creating the learning method for the user is the same as in the first embodiment. That is, in FIG. 4 and FIG. 5B, "practice" means "learning of educational subjects." The activity contents may include information on whether the user uses the learning method in combination with lessons at school, preparatory school, or correspondence course, for example. The current ability may include not only regular learning (practice history) but also scores of external academic tests and mock examinations. When the learning methods, texts, and so forth are provided by educational institutions, such as schools, the ability may include results of regular examinations conducted by those educational institutions. These pieces of information are stored in the user information 7321.

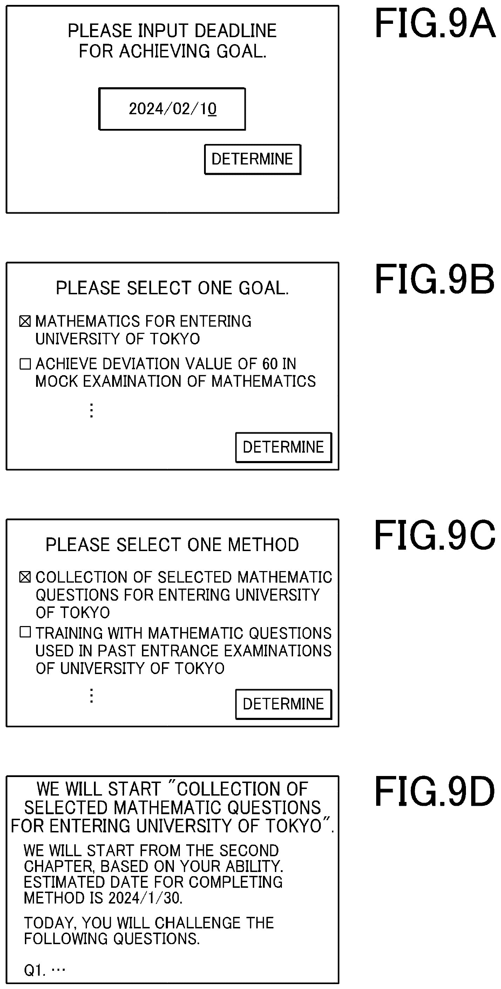

In setting the learning method, the display 45 sequentially displays setting screens as illustrated in FIG. 9A to FIG. 9D as an example. First, the screen for inputting the deadline for achieving the goal is displayed, as illustrated in FIG.9A. When the user inputs the year and date and selects the determination button with the operation receiver 46, goals that correspond to the deadline for achieving the goal, the current ability, and learning states are searched for and displayed in a list, as illustrated in FIG. 9B.

When the user selects one of the goals and selects the determination button by input operations, learning methods that correspond to the goal are found. The display 45 displays multiple types of learning methods in a list, as illustrated in FIG. 9C. Herein, the date at which the user is expected to achieve the goal may also be displayed, as in the first embodiment. When the user selects one of the learning methods and selects the determination button by inputting operations with the operation receiver 46, learning contents corresponding to the selected learning method are determined, and the learning start screen is displayed, as illustrated in FIG. 9D. The learning start screen may show the selected learning method together with the date at which the user is expected to achieve the goal.

In the user information 7321, the review history and execution history of contents provided to the user according to the above learning method are stored, as illustrated in FIG. 8B. Questions may be stored along with information on whether the user gave a correct answer. As described above, in a case where the contents (questions) provided in the past are repetitively reviewed and answered, the review may be added to the history each time. Further, cumulative viewing time and response time may be calculated for each of the viewed contents.

A range of necessary knowledge and a necessary degree of understanding for answering each question may be set and stored in the learning data 7322, as illustrated in FIG. 8C. The illustrated example shows that the user needs a precise understanding of integration along with knowledge of differentiation, trigonometric functions, and exponential/logarithmic functions to correctly answer the question. When a correct answer is given, the degree of understanding of each unit can be added as a parameter or updated to the maximum value. When a correct answer is not given, the information on the question may be used for suggesting contents to be reviewed. The numerical values expressing the degree of understanding are set for convenience and can be determined according to the level expected to be acquired in a target group (e.g., high school entrance examinations, university entrance examinations).

Further, in the learning data 7322, required correct answer rates for questions provided by each learning method may be stored, as illustrated in FIG. 8D. In many cases of preparation for examinations, an examinee is not required to correctly answer all questions but need to answer a certain level of questions on average. Especially, the examinee needs the ability to certainly solve easy questions to earn scores. Conditions for completing the learning method may include obtaining the ability to give correct answers to a certain level of questions required for achieving the goal, based on weighting of questions by the correct answer rate.

As described above, according to the embodiment, the information processing method to be performed by a computer includes obtaining a target period for the user to achieve his/her goal; determining a goal, based on the target period and the current ability of the user; and determining the practice method, based on the goal and the target period. The information processing method can prepare a practice plan, based on not only short-term improvements but also a medium-term strategy for achieving the goal by the deadline. Thus, the information processing method can propose effective practice to the user.

Further, the information processing method may output multiple candidates for the practice method, based on the target period and the goal, and may determine the practice method by obtaining the result of selection from the output candidates. Thus, the information processing method does not force a single practice method but provides multiple candidates for the practice method so that the user can select a practice method that seems suitable for him/her.

Further, based on the current ability of the user, the target period, and the goal, the information processing method may calculate the estimated period for the user to complete practice according to a candidate for the practice method, and may output the calculated estimated period in association with the candidate. Thus, the user can consider how much time the user has for completing practice and can estimate whether he/she can complete the practice, based on his/her motivation and securable practice time.

Further, when the user selects a practice method from the output candidates for the practice method and the practice method is determined, the information processing method may output the determined practice method and the above estimated period instead of outputting the candidates for the practice method. Thus, the user can check whether he/she has made a right selection and can start practice in accordance with the determined schedule toward the goal.

Further, the information processing method may arrange the candidates for the practice candidate, based on the difference between the estimated period and the target period, and may output the candidates in the arranged order. Thus, the user can consider whether each candidate is appropriate in the proper order, based on the user's ability and the target period.

Further, the information processing method may refer to the storage 73 that stores information on multiple practice methods and may output multiple candidates for the practice method. By referring to the database that stores beforehand information on numerous practice methods, it is possible to certainly extract suitable practice method candidates for the user.

Further, the current ability of the user may be identified, based on the practice history of the user. Based on the contents of daily practice, it is possible to grasp objective information, such as the running speed at which the user runs a certain distance, or whether the user has improved his/her ability or has stagnated, for example. Based on such objective information, the information processing method can provide more appropriate practice method candidates to the user.

Further, the practice may include learning of educational subjects; and the practice method may include a learning method for learning the educational subjects. That is, the practice method is effectively applicable to brain training as well as physical training including sports. Thus, the user can engage in learning training by his/her own at an appropriate pace tailored for the user, without relying on general instructions by teachers or lecturers at schools.

Further, the information processing method may output multiple candidates for the goal, based on the target period and the current ability of the user, and may set the goal by obtaining the result of selection from the output candidates. Thus, the user may be allowed to select the goal as well as the practice method. By providing a goal that is realistically achievable based on the deadline for achieving a goal, the information processing method allows the user to set a goal in consideration of realistic achievability.

Further, the current ability of the user may be identified by searching the database 732 stored in the storage 73, based on the identification information of the user, and by obtaining the practice history of the user. With the database 732 that stores and manages the practice history of many users, it is possible to easily provide suitable practice methods for the respective users.

Further, the information processing method of this embodiment may create a practice plan, based on the determined practice method, and may output the practice plan by displaying the practice plan on the display 45 or the display 75 or may output the practice plan as a document file. Thus, a practice plan corresponding to the practice method suitable for the user can be easily viewed by the user. Thus, the user can continue appropriate practice toward his/her goal.

Further, the program 731 of this embodiment causes the computer to function as: (1) the obtaining means that obtains a target period for goal achievement of a user; (2) the setting means that sets a goal, based on the target period and current ability of the user; and (3) the determining means that determines a practice method, based on the goal and the target period. With the program 731, it is possible to easily propose a goal for improving the level of the user and a practice method corresponding to the goal without special devices, as long as necessary data can be obtained.

Further, the server device 7 as the information processing apparatus of the embodiment includes the controller 71. The controller 71 obtains the target period for the user to achieve his/her goal. The controller 71 sets a goal, based on the target period and the current ability of the user. The controller 71 determines the practice method according to the goal and the target period. According to such a procedure, the server device 7 can propose an appropriate goal and practice method for the user and guide the user to improve his/her ability.

The above embodiments are not intended to limit the present disclosure and can be variously modified. For example, although the goal and the practice method are determined in order in the above embodiments, the present disclosure is not limited to this. Since the realistic range of goals is not so wide, the range of goals and the range of practice methods can be determined at the same time. For another example, the determined goal may be adjusted to some degree depending on the practice method candidate.

Although the deadline is fixed on a specific day in the above, the present disclosure is not limited to this. For example, consider a case where there is time until the next competition/examination or a case where the user has not determined which competition/examination to participate next and wishes to set a goal in an appropriate period. In such cases, a range of time periods may be set as the target period.

Although a single estimated period, during which the user is estimated to complete practice, is output for the practice method in the above description, the present disclosure is not limited to this. A range of estimated periods may be output that can be shortened or extended by adjusting the amount of practice per day.

Although the candidates are output in order of proximity to the target period in the above description, the present disclosure is not limited to this. The candidates may be output in the order of easiness of achieving the goal. In such a case, the candidates may be output with colored backgrounds or reference markers so that the user can recognize the candidate most suitable for the target period at a glance.

Although the current ability of the user is obtained based on the practice history in the above description, it is possible that a necessary practice history for identification is not stored. In such a case, running by the user may be measured once or the user may be tested with test questions for grasping the current ability of the user. Based on the result of the measurement/test, the ability of the user may be identified. For another example, the user may be invited to input data, such as the recorded time at a competition, as a minimum piece of information. Based on the recorded time, the ability may be estimated.

Although the practice method for achieving the goal in improving running ability and academic performance is provided in the above description, the target of the practice method is not limited to these. For example, the present disclosure may be applicable to improvement of ability in various exercises and board games.

Although the server device 7 or the electronic device 4 performs the plan creating process in the above description, the process may be distributed and performed by multiple information processing devices (controllers).

In the above description, the storage 73 is a nonvolatile memory, such as an HDD or a flash memory, as an example of a computer-readable medium for storing the program 731 related to creating a practice plan in the present disclosure. However, the computer-readable medium is not limited to these. The computer-readable medium can be a different type of nonvolatile memory, such as a magnetoresistive random access memory (MRAM), or a portable recording medium, such as a CD-ROM or a DVD. Further, a carrier wave may be used as a medium to provide data of the program of the present disclosure via a communication line.

The detailed configurations, contents and orders of the steps in the processes, and so forth shown in the above embodiment can be appropriately modified without departing from the scope of the present disclosure. It is intended that the scope of the present invention includes the scope of the invention described in the scope of the claims and the scope of equivalents thereof.

## Claims

1. An information processing method to be performed by a computer (4, 7, 4a, 7a), comprising:
obtaining a target period for goal achievement of a user;
setting a goal, based on the target period and current ability of the user; and
determining a practice method, based on the goal and the target period.

2. The information processing method according to claim 1, wherein:
based on the target period and the goal, the computer outputs a candidate for the practice method, and
the computer determines the practice method by obtaining a result of selection from the output candidate.

3. The information processing method according to claim 2, wherein:
based on the current ability of the user, the target period, and the goal, the computer calculates an estimated period for the user to complete practice according to the candidate, and
the computer outputs the estimated period in association with the candidate.

4. The information processing method according to claim 3, wherein:
in response to the user selecting the practice method from the output candidate, the computer determines the practice method, and
the computer outputs the determined practice method and the estimated period instead of outputting the candidate for the practice method.

5. The information processing method according to claim 3, wherein:
the computer arranges the candidate for the practice method, based on a difference between the estimated period and the target period, and
the computer outputs the candidate in the arranged order.

6. The information processing method according to claim 2, wherein the computer refers to a storage (73) that stores information on multiple practice methods and outputs multiple candidates for the practice method.

7. The information processing method according to claim 1, wherein the computer identifies the current ability of the user, based on a practice history of the user.

8. The information processing method according to claim 1, wherein:
based on the target period and the current ability of the user, the computer outputs multiple candidates for the goal, and
the computer sets the goal by obtaining a result of selection from the output candidates.

9. The information processing method according to claim 1, wherein:
the practice includes learning of an educational subject, and
the practice method includes a learning method for learning the educational subject.

10. The information processing method according to claim 7, wherein:
the computer obtains the practice history of the user by referring to a database (732) stored in a storage (73), based on identification information of the user.

11. The information processing method according to claim 1, wherein:
the computer creates a practice plan, based on the determined practice method, and
the computer outputs the practice plan by displaying the practice plan on a display (45, 75) or outputs the practice plan as a document file.

12. A program (731) that causes a computer (4, 7, 4a, 7a) to:
obtain a target period for goal achievement of a user;
set a goal, based on the target period and current ability of the user; and
determine a practice method, based on the goal and the target period.

13. An information processing device (4, 7, 4a, 7a) that includes a processor (71, 41), wherein the processor
obtains a target period for goal achievement of a user,
sets a goal, based on the target period and current ability of the user, and
determines a practice method, based on the goal and the target period.

14. An information output system (100, 100a) comprising:
a server device (7, 7a) that includes a processor (71); and
an electronic device (4, 4a) that is communicably connected to the server device over a network,
wherein the processor
obtains a target period for goal achievement of a user,
sets a goal, based on the target period and current ability of the user, and
determines a practice method, based on the goal and the target period.
